# DEMANDE DE BREVET EUROPEEN

(11) **EP 0 962 233 A1**
(43) Date de publication de la demande: **08.12.1999**
(21) Numéro de dépôt: 98440117.4
(22) Date de dépôt: 03.06.1998
(51) Int. Cl.: A61N 1/04, A61N 1/30

(54) **Dispositif d'electrode pour electrotherapie**

(71) Demandeur: Sport-Elec S.A., 27520 Bourgtheroulde (FR)
(72) Inventeur: Coral, Hervé, F-27350 Le Landin (FR); Herbert, Patrick, F-14540 Garcelles (FR)
(74) Mandataire: Arbousse-Bastide, Jean-Claude Philippe

(57) **Abrégé**

Dispositif d'électrode pour électrothérapie destiné à être utilisé avec un générateur de courant unidirectionnel en émission fractionnée ou biphasée.

Il comporte un élément conducteur (1) recouvert d'une couche (3) d'une matière adhérente du type hydrogel, ainsi que des moyens (2) permettant, lors de son utilisation, d'alimenter en eau ladite matière adhérente.

Il comporte un élément (2) fait d'un matériau absorbant destiné à être imbibé d'eau, disposé entre l'élément conducteur (1) et la couche (3) de matière adhérente de type hydrogel, et qui constitue un réservoir d'eau.

## Description

La présente invention a pour objet un dispositif d'électrode pour électrothérapie destiné à être utilisé avec un générateur de courant unidirectionnel en émission fractionnée ou biphasée.

L'électrothérapie consiste à utiliser l'électricité comme moyen thérapeutique, le plus fréquemment pour réaliser des stimulations musculaires, ou, dans le cas d'une ionothérapie, pour faire pénétrer dans l'organisme des produits actifs ionisés.

Les dispositifs d'électrode utilisés par les thérapeutes se présentent généralement sous la forme d'un élément qui comprend un matériau absorbant, et des moyens de fixation de sangles ou analogues, permettant de ceinturer le corps pour assurer l'application dudit élément.

Les dispositifs d'électrode adaptés aux générateurs à usage domestique consistent généralement en un élément conducteur souple dont la face destinée à venir au contact de la peau est recouverte d'une couche faite d'une matière permettant d'une part le passage du courant et d'autre part une adhérence à la peau.

La matière qui recouvre l'élément conducteur consiste généralement en un hydrogel qui nécessite d'être régénéré par un apport en eau avant chaque utilisation. En pratique, on enlève une feuille de protection qui recouvre la couche d'hydrogel, puis on humecte celle-ci d'eau, et on attend un certain temps afin de permettre l'absorption complète de l'eau par l'hydrogel.

Les manipulations que nécessite cette opération d'apport en eau sont peu commodes, car d'une part elles ne peuvent pas être réalisées n'importe où, et d'autre part, après enlèvement de la feuille de protection, l'électrode est difficilement maniable.

De plus, pour conserver son pouvoir adhésif et une faible résistivité, l'hydrogel doit garder un degré d'hydrométrie constant. Or, au contact de la peau et du fait de l'échauffement créé par le passage du courant, l'hydrogel se dessèche.

L'augmentation de la résistivité due au dessèchement de l'hydrogel entraîne une diminution de la puissance transmise par le générateur vers la peau au travers du dispositif d'électrode, ce que l'utilisateur va chercher à compenser en augmentant la puissance du générateur. Cette compensation est très mal ressentie, car la sensation du courant devient très vite désagréable du fait que certains points, moins "secs" que d'autres, privilégient le passage du courant.

La présente invention a pour but de proposer un dispositif d'électrode permettant de remédier à ces divers inconvénients. Il permet d'autre part d'être utilisé en ionothérapie.

Le dispositif d'électrode selon l'invention comporte un élément conducteur recouvert d'une couche d'une matière adhérente du type hydrogel, et il se caractérise essentiellement en ce qu'il comporte des moyens permettant, lors de son utilisation, d'alimenter en eau ladite matière adhérente.

Selon un mode de réalisation particulier du dispositif d'électrode selon l'invention, il comporte un élément fait d'un matériau absorbant destiné à être imbibé d'eau, disposé entre l'élément conducteur et la couche de matière adhérente de type hydrogel, et qui constitue un réservoir d'eau.

Lors de l'utilisation du dispositif d'électrode selon l'invention, la matière adhérente de type hydrogel puise l'eau dans l'élément absorbant, en sorte qu'elle demeure à un degré d'hydrométrie sensiblement constant.

Selon une caractéristique additionnelle du dispositif selon l'invention, la couche de matière adhérente de type hydrogel est percée, au droit de l'élément absorbant, d'au moins un trou permettant d'imbiber d'eau ledit élément absorbant avant utilisation.

Selon une autre caractéristique additionnelle du dispositif selon l'invention, la couche de matière adhérente de type hydrogel est recouverte d'une feuille de protection présentant en regard du ou des trous pratiqués dans ladite couche de trous permettant, avant utilisation, d'imbiber d'eau l'élément absorbant sans avoir à enlever ladite feuille de protection.

Selon un autre mode de réalisation du dispositif d'électrode selon l'invention, il comporte un élément fait d'un matériau absorbant constituant un réservoir d'eau, disposé contre l'élément conducteur et inséré dans un espace ménagé dans la couche de matière adhérente de type hydrogel.

Selon une caractéristique additionnelle de cet autre mode de réalisation du dispositif selon l'invention la couche de matière adhérente de type hydrogel est recouverte d'une feuille de protection présentant, en regard de l'espace où loge l'élément absorbant, un trou permettant à ce dernier d'être remplacer et/ou d'être alimenté en eau sans nécessiter l'enlèvement de ladite feuille de protection.

Conformément à l'invention, l'eau destinée à imbiber l'élément absorbant est additionnée d'un produit actif ionisé.

Les avantages et les caractéristiques de la présente invention ressortiront plus clairement de la description qui suit et qui se rapporte au dessin annexé, lequel en représente plusieurs modes de réalisation non limitatifs.

Dans le dessin annexé:
- la figure 1 représente une vue en perspective et en éclaté d'un premier mode de réalisation du dispositif d'électrode selon l'invention.
- la figure 2 représente une vue perspective et en éclaté d'un second mode de réalisation du même dispositif.

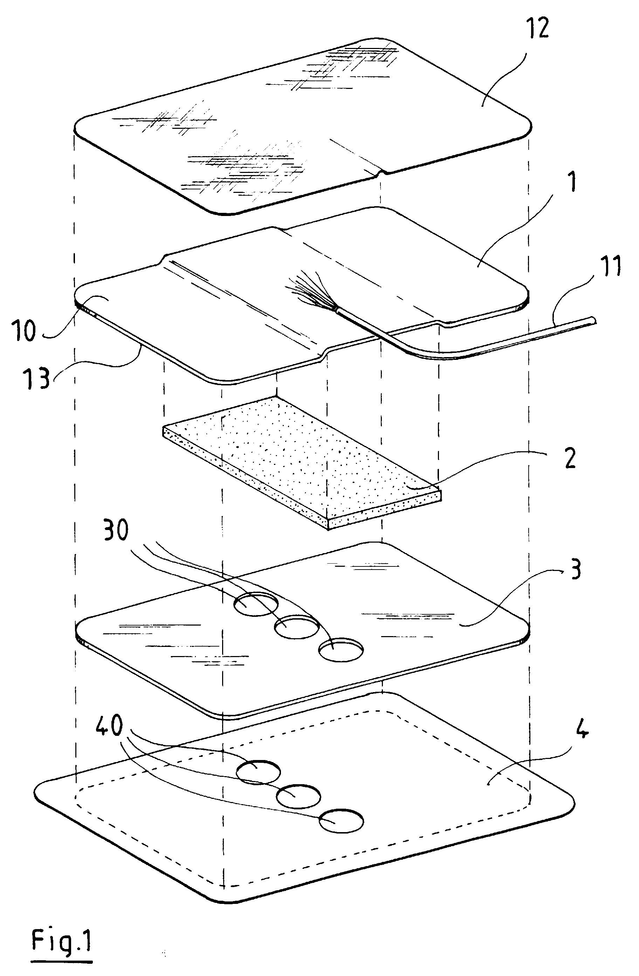

Si on se réfère à la figure 1, on peut voir qu'un dispositif d'électrode selon l'invention comporte un élément conducteur 1, qui de préférence est réalisé en carbone, au contact de la face supérieure 10 duquel est disposé un fil conducteur 11 destiné à être connecté à un générateur de courant, non représenté.

On notera que la face supérieure 10 et le fil conducteur 11 sont recouverts d'une pièce 12 de matière isolante qui peut être réalisée dans un matériau du type non-tissé.

Un élément 2, fait d'un matériau absorbant du type feutre par exemple, est positionné contre la face inférieure 13 de l'élément conducteur 1, maintenu par une couche 3 d'une matière adhérente à la peau, telle qu'un hydrogel.

On notera que l'élément 2 est de dimensions inférieures à celle de l'élément conducteur 1, et qu'il est disposé dans la région médiane de ce dernier.

La couche 3 est recouverte d'une feuille de protection 4 présentant des dimensions supérieures pour en faciliter l'enlèvement.

La couche 3 présente trois trous 30 pratiqués au droit de l'élément absorbant 2, tandis que la feuille de protection 4 présente également trois trous 40 réalisés en regard des trous 30, en sorte que l'élément absorbant 3 soit accessible au travers de ces trous 40 et 30.

En pratique, on imbibe d'eau l'élément absorbant 2, ce qui peut être réalisé de manière aisé en passant le dispositif d'électrode sous un robinet. Puis, par capillarité la matière adhérente de type hydrogel puise dans l'élément absorbant l'eau permettant de la régénérer. Enfin on enlève la feuille de protection et on place le dispositif d'électrode sur la peau.

Durant l'opération d'électrothérapie, la matière adhérente de type hydrogel continue à puiser de l'eau dans l'élément absorbant 2 en sorte qu'elle ne dessèche pas et que son degré d'hydrométrie demeure sensiblement constant.

Par rapport aux électrodes connues, le dispositif d'électrode selon l'invention nécessite donc ainsi moins de manipulations pour sa mise en place, et les manipulations sont plus aisées.

D'autre part, il est possible d'incorporer à l'eau un produit actif ionisé permettant de réaliser une ionothérapie.

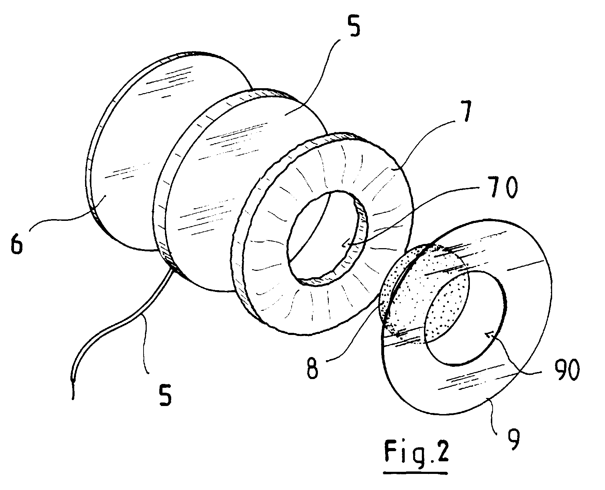

Si on se réfère maintenant à la figure 2, on peut voir que dans un second mode de réalisation du dispositif selon l'invention, celui-ci comporte également un élément conducteur 5 et un fil conducteur 50 recouvert un élément isolant 6, une couche 7 faite d'une matière adhérente du type hydrogel, un élément absorbant 8 et une feuille de protection 9.

La couche 7 de matière adhérente de type hydrogel présente un trou 70 dans lequel est placé l'élément absorbant 8 qui est de forme complémentaire, tandis que la feuille de protection 9 présente un trou 90 en regard du trou 70.

L'élément absorbant 8 est au contact de l'élément conducteur 5, et par ses bords 80 au contact de la couche 7 de matière adhérente de type hydrogel afin de permettre l'alimentation de celle-ci en eau.

Le dispositif d'électrode selon ce second mode de réalisation s'utilise comme le premier, par contre, il autorise le remplacement de l'élément absorbant 8 par un autre.

Cette disposition présente un avantage lors de la réalisation d'une ionothérapie. En effet lors d'ionisations successives il est important que les électrodes qui ont servi à la diffusion d'un produit ionisé ne soient pas réutilisées pour la diffusion d'un produit de polarité différente. Aussi, la possibilité d'enlèvement et de remplacement de l'élément absorbant 8 permet d'utiliser de tels éléments à usage unique.

De plus, afin d'éviter des erreurs de concentration de substance ionisée, les éléments absorbants peuvent être conditionnés pré-imprégnés de produit actif ionisé, prêts à l'emploi, en sorte que le produit actif y soit parfaitement dosé, ce qui permet la réalisation de ionothérapies par le grand public.

D'autre part, la notice d'utilisation de l'élément absorbant peut figurer sur le conditionnement de celui-ci. Elle peut ainsi comporter la nature du produit, les lieux d'application des électrodes, la nature du courant à utiliser et le temps d'exposition.

Le dispositif d'électrode selon l'invention peut être utilisé par le grand public, mais également par les thérapeutes.

## Revendications

1. Dispositif d'électrode pour électrothérapie destiné à être utilisé avec un générateur de courant unidirectionnel en émission fractionnée ou biphasée, comportant un élément conducteur (1; 5) recouvert d'une couche (3; 7) d'une matière adhérente du type hydrogel, caractérisé en ce qu'il comporte des moyens (2; 8) permettant, lors de son utilisation, d'alimenter en eau ladite matière adhérente.

2. Dispositif selon la revendication 1 caractérisé en ce qu'il comporte un élément (2) fait d'un matériau absorbant destiné à être imbibé d'eau, disposé entre l'élément conducteur (1) et la couche (3) de matière adhérente de type hydrogel, et qui constitue un réservoir d'eau.

3. Dispositif selon la revendication 2 caractérisé en ce que la couche (3) de matière adhérente de type hydrogel est percée, au droit de l'élément absorbant (2), d'au moins un trou (30) permettant d'imbiber d'eau ledit élément absorbant (2) avant utilisation.

4. Dispositif selon la revendication 3, caractérisé en ce que la couche (3) de matière adhérente de type hydrogel est recouverte d'une feuille de protection (4) présentant en regard du ou des trous (30) pratiqués dans ladite couche (3) de trous (40) permettant d'imbiber d'eau l'élément absorbant (2) sans avoir à enlever ladite feuille de protection (4).

5. Dispositif selon l'une quelconque des revendications, précédentes caractérisé en ce que l'eau destinée à imbiber l'élément absorbant (2) est additionnée d'un produit actif ionisé.

6. Dispositif selon la revendication 1, caractérisé en ce qu'il comporte un élément (8) fait d'un matériau absorbant constituant un réservoir d'eau, disposé contre l'élément conducteur (50) et inséré dans un espace (70) ménagé dans la couche (7) de matière adhérente de type hydrogel.

7. Dispositif selon la revendication 6, caractérisé en ce que la couche (7) de matière adhérente de type hydrogel est recouverte d'une feuille de protection (9) présentant, en regard de l'espace (70) où loge l'élément absorbant (7), un trou (90) permettant à ce dernier d'être remplacer et/ou d'être alimenté en eau sans nécessiter l'enlèvement de ladite feuille de protection (9).

8. Dispositif selon la revendication 6 ou la revendication 7, caractérisé en ce que l'eau destinée à imbiber l'élément absorbant (8) est additionnée d'un produit actif ionisé.

9. Dispositif selon la revendication 8 caractérisé en ce que l'élément absorbant (8) est conditionné pré-imprégné d'un produit actif ionisé.
